# EUROPEAN PATENT SPECIFICATION

(11) **EP 4 395 464 B1**
(45) Date of publication and mention of the grant of the patent: **06.08.2025**
(21) Application number: 21961274.4
(22) Date of filing: 30.12.2021
(51) Int. Cl.: A61N 5/06, H05B 45/20

(54) **RHYTHM SPECTRUM MODULATION METHOD**
VERFAHREN ZUR MODULATION DES RHYTHMUSSPEKTRUMS
PROCÉDÉ DE MODULATION DU SPECTRE DE RYTHME

(30) Priority: 18.10.2021 CN 202111210303; 18.10.2021 CN 202111209089
(43) Date of publication of application: 03.07.2024
(73) Proprietor: Foshan Electrical and Lighting Co., Ltd, Foshan, Guangdong 528000 (CN)
(72) Inventor: DING, Wenchao, Foshan, Guangdong 528000 (CN); ZHU, Yiguang, Foshan, Guangdong 528000 (CN); WEI, Bin, Foshan, Guangdong 528000 (CN); CHENG, Xiaofang, Foshan, Guangdong 528000 (CN)
(74) Representative: Sun, Yiming
(86) International application number: PCT/CN2021/143144
(87) International publication number: WO 2023/065536

(56) References cited:
- CN-A- 110 024 141
- CN-A- 111 162 153
- CN-A- 111 919 737
- CN-A- 112 255 783
- CN-A- 112 255 785
- JP-A- 2013 175 679
- US-A1- 2004 093 045
- US-A1- 2021 164 624
- US-A1- 2021 176 837

## Description

### TECHNICAL FIELD

The present disclosure relates to the field of luminescent spectra, and in particular to a method for modulating a rhythmic spectrum.

### BACKGROUND

Indoor lighting environments have a huge influence on health of human bodies. Evidences show that lighting affects visible, physiological and behavioral functions of the human being. The influence of the lighting is associated with intrinsically photosensitive retinal ganglion cells (ipRGCs). Light signals are transmitted from the ipRGCs to the suprachiasmatic nucleus (SCN), thereby adjusting and controlling biological clocks and various physiological activities of the human bodies. If the human bodies receive appropriate light stimulation every day, circadian rhythms in the human bodies can be corrected desirably. Otherwise, the balanced state in the human bodies is disturbed to cause great hazards. The ipRGCs are activated by internal photosensitive proteins called melanopsin. Upon this, amelanopic spectral efficiency function is established to calculate a "melanopic luminous flux", which is then improved as an equivalent melanopic lux (EML). Generally, the EML is used as an evaluation standard to design a rhythmic spectrum.

The rhythm effect of the lighting is mainly affected by a spectral composition, a light intensity, lighting time, etc. It is mostly adjusted by controlling the light intensity and a color temperature. This method is inevitable to affect visual changes, such as the color temperature and a color rendering index (CRI), to reduce a visual effect. Hence, it is difficult to adjust the rhythmic stimulation effect and the visual effect at the same time.

In addition, according to the Shanghai Group Standard T/SIEATA *Ranking Evaluation for Classroom Lighting Quality in Primary and Middle Schools,* the class AAAAArequires that an EML value is greater than or equal to 200 and an average illuminanceis greater than or equal to 500 Ix in daytime, and an EML value is less than or equal to130 and an average illuminanceis greater than or equal to 300 Ix in nighttime.

Publication US 2021/164624 A1 describes a system for emitting light to improve cognitive performance. **SUMMARY**

A technical problem to be solved by the present disclosure is to provide a method for modulating a rhythmic spectrum, to achieve a high CRI and an appropriate rhythmic stimulation level.

To solve the above technical problem, the present disclosure provides a method for modulating a rhythmic spectrum, including:
(1) providing a white light-emitting diode (LED), a blue LED, a green LED and a blue-green LED on a substrate; and
(2) adjusting a luminous flux of each of the white LED, the blue LED, the green LED and the blue-green LED to a preset range, thereby obtaining a daytime or nighttime rhythmic spectrum with a preset luminous flux,
where in response to a daytime lighting mode, the luminous flux of the white LED accounts for 62.32-75.9% of the preset luminous flux, the luminous flux of the blue LED accounts for 1.74-1.91% of the preset luminous flux, the luminous flux of the green LED accounts for 1.5-20.27% of the preset luminous flux, and the luminous flux of the blue-green LED accounts for 15.5-22.8% of the preset luminous flux; and in response to a nighttime lighting mode, the luminous flux of the white LED accounts for 61.8-69.3% of the preset luminous flux, the luminous flux of the blue LED accounts for 0.72-0.88% of the preset luminous flux, the luminous flux of the green LED accounts for 28.6-37.31% of the preset luminous flux, and the luminous flux of the blue-green LED accounts for 0-1.8% of the preset luminous flux.

As an improvement to the above technical solution, step (2) includes:
(2.1) adjusting the luminous flux of each of the white LED, the blue LED, the green LED and the blue-green LED, where when the luminous flux of one LED is adjusted, other LEDs are turned off; and
(2.2) turning on the white LED, the blue LED, the green LED and the blue-green LED at the same time to obtain the rhythmic spectrum with the preset luminous flux in the daytime or nighttime lighting mode.

As an improvement to the above technical solution, the white LED has a color temperature of 2,600-3,300 K.

As an improvement to the above technical solution, the blue LED has a peak wavelength of 450-465 nm.

As an improvement to the above technical solution, the green LED has a peak wavelength of 540-560 nm. As an improvement to the above technical solution, the blue-green LED has a peak wavelength of 490-500 nm.

As an improvement to the above technical solution, the white LED has the color temperature of 2,700 K, and the blue LED has the peak wavelength of 455 nm.

As an improvement to the above technical solution, the green LED has the peak wavelength of 550 nm, and the blue-green LED has the peak wavelength of 495 nm.

As an improvement to the above technical solution, in response to the daytime lighting mode, the luminous flux of the white LED accounts for 62.32-75.9% of the preset luminous flux, the luminous flux of the blue LED accounts for 1.74-1.91% of the preset luminous flux, the luminous flux of the green LED accounts for 1.5-20.27% of the preset luminous flux, and the luminous flux of the blue-green LED accounts for 15.5-22.8% of the preset luminous flux; and in response to the nighttime lighting mode, the luminous flux of the white LED accounts for 61.8-69.3% of the preset luminous flux, the luminous flux of the blue LED accounts for 0.72-0.88% of the preset luminous flux, the luminous flux of the green LED accounts for 28.6-37.31% of the preset luminous flux, and the luminous flux of the blue-green LED accounts for 0-1.8% of the preset luminous flux.

The present disclosure has the following beneficial effects:
The present disclosure uses a white LED channel, a blue LED channel, a green LED channel and a blue-green LED channel. By adjusting the luminous flux of each channel, the present disclosure obtains the daytime rhythmic spectrum with strong rhythmic stimulation and a high CRI or the nighttime rhythmic spectrum with weak rhythmic stimulation and a high CRI. The modulation method is simple and easy. While ensuring the high CRI and general lighting, the present disclosure effectively reduces the rhythmic stimulation level in nighttime lighting.

### BRIEF DESCRIPTION OF THE DRAWINGS

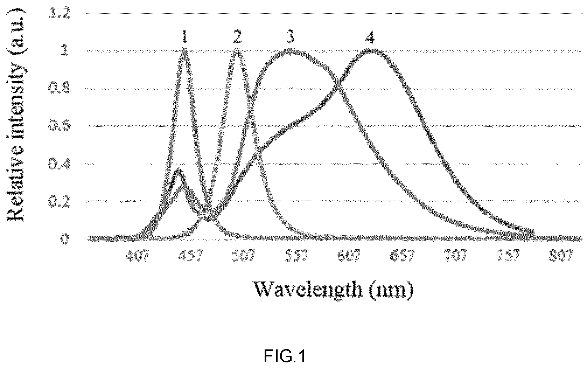

FIG. 1 illustrates individual spectra of four LEDs according to Embodiments 1-4 and Comparative Embodiments 1-10 of the present disclosure, 1 being a blue LED with a peak wavelength of 455 nm, 2 being a blue-green LED with a peak wavelength of 495 nm, 3 being a green LED with a peak wavelength of 550 nm, and 4 being a white LED with a color temperature of 2,700 K;

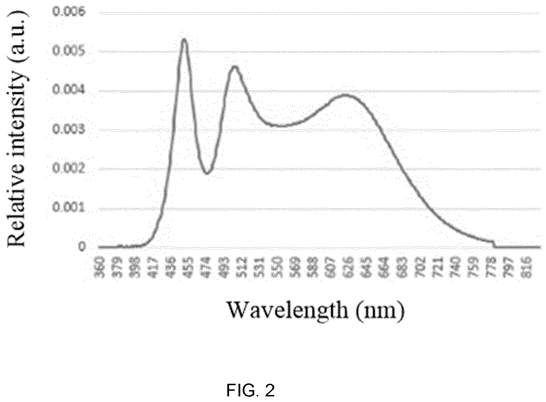

FIG. 2 illustrates a daytime rhythmic spectrum obtained in Embodiment 1 of the present disclosure;

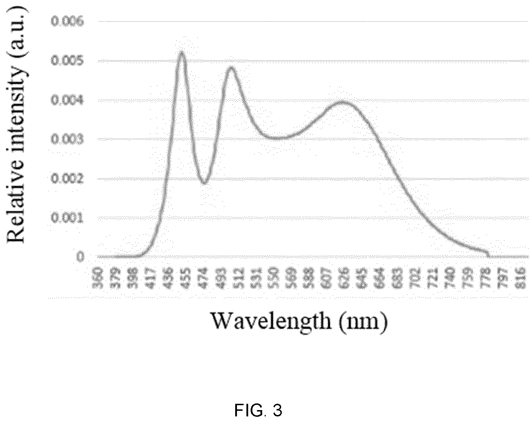

FIG. 3 illustrates a daytime rhythmic spectrum obtained in Embodiment 2 of the present disclosure;

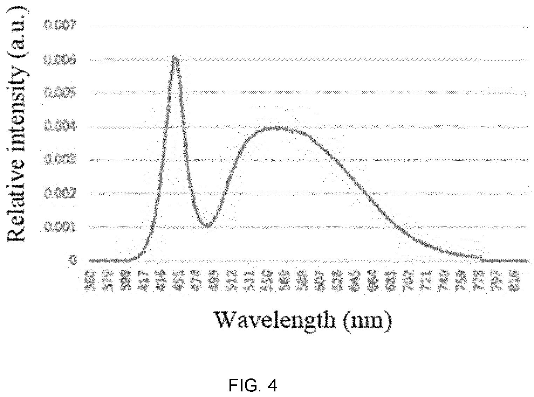

FIG. 4 illustrates a daytime rhythmic spectrum obtained in Comparative Embodiment 1 of the present disclosure;

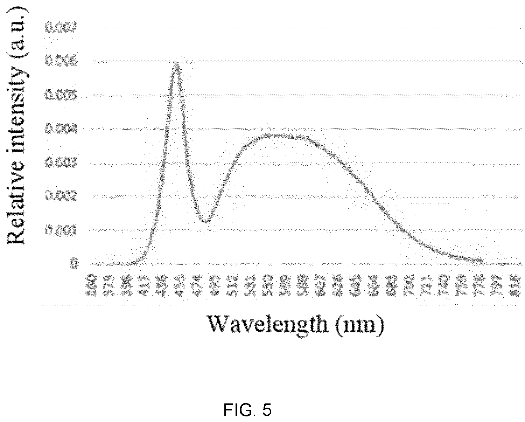

FIG. 5 illustrates a daytime rhythmic spectrum obtained in Comparative Embodiment 2 of the present disclosure;

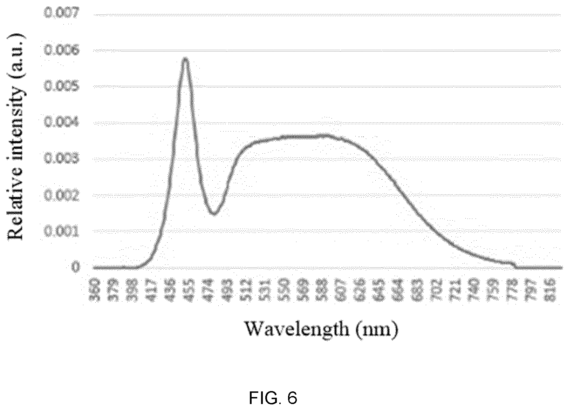

FIG. 6 illustrates a daytime rhythmic spectrum obtained in Comparative Embodiment 3 of the present disclosure;

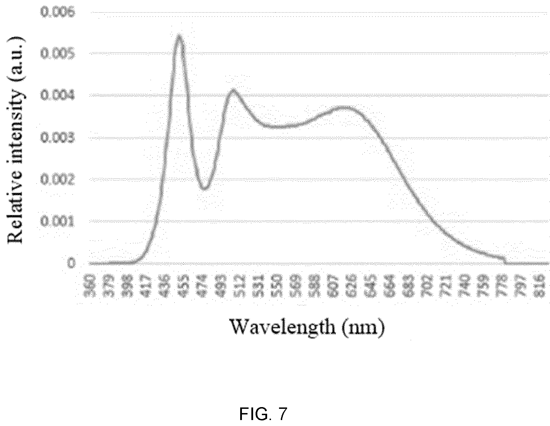

FIG. 7 illustrates a daytime rhythmic spectrum obtained in Comparative Embodiment 4 of the present disclosure;

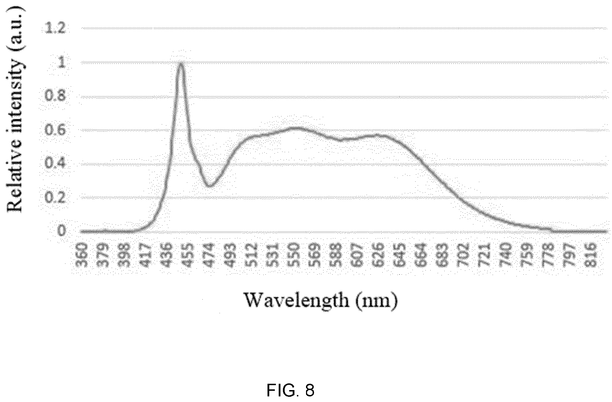

FIG. 8 illustrates a daytime rhythmic spectrum obtained in Comparative Embodiment 5 of the present disclosure;

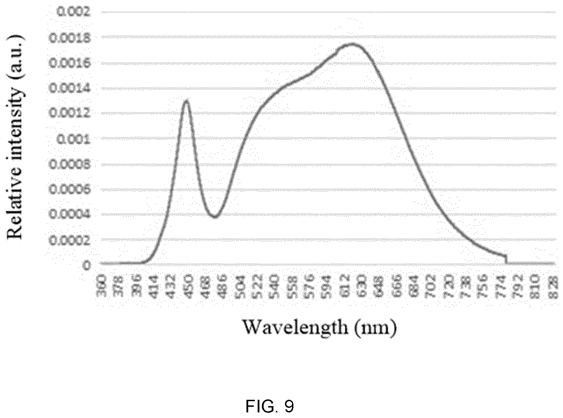

FIG. 9 illustrates a nighttime rhythmic spectrum obtained in Embodiment 3 of the present disclosure;

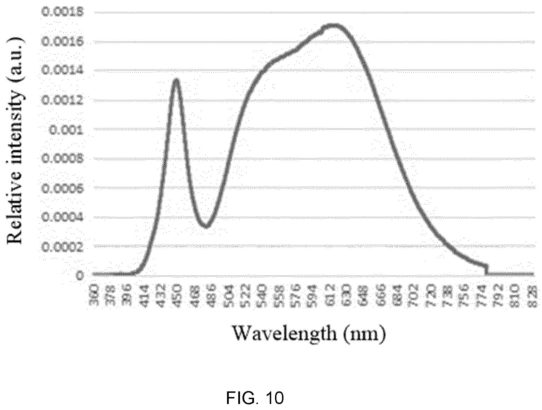

FIG. 10 illustrates a nighttime rhythmic spectrum obtained in Embodiment 4 of the present disclosure;

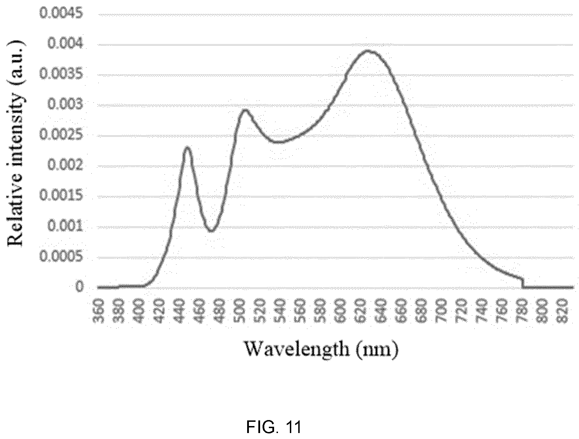

FIG. 11 illustrates a nighttime rhythmic spectrum obtained in Comparative Embodiment 6 of the present disclosure;

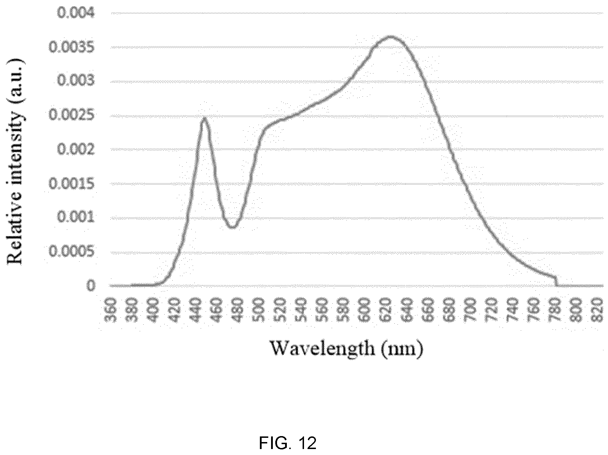

FIG. 12 illustrates a nighttime rhythmic spectrum obtained in Comparative Embodiment 7 of the present disclosure;

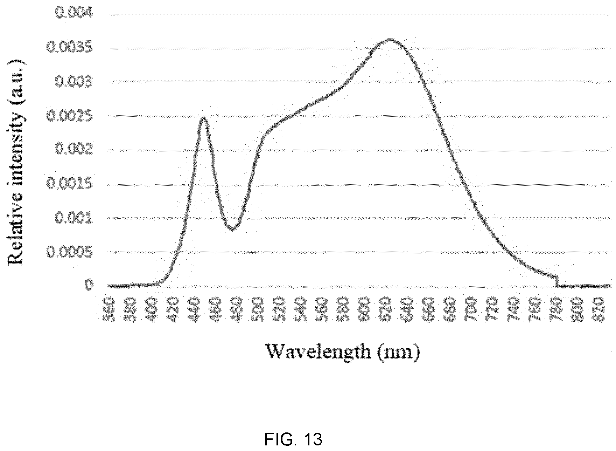

FIG. 13 illustrates a nighttime rhythmic spectrum obtained in Comparative Embodiment 8 of the present disclosure;

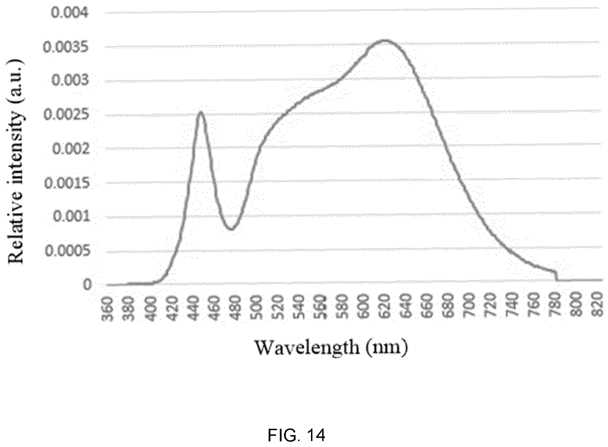

FIG. 14 illustrates a nighttime rhythmic spectrum obtained in Comparative Embodiment 9 of the present disclosure; and

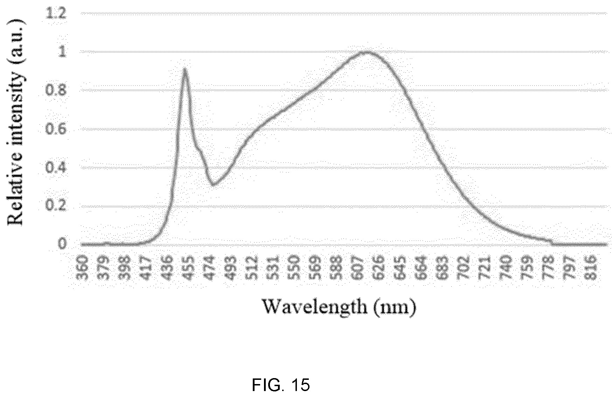

FIG. 15 illustrates a nighttime rhythmic spectrum obtained in Comparative Embodiment 10 of the present disclosure.

### DETAILED DESCRIPTION

To make the objectives, technical solutions, and advantages of the present disclosure clearer, the present disclosure will be further described in detail below in combination with specific embodiments.

A high rhythmic stimulation effect and a high visual effect (CRI) are unachievable to the conventional method for modulating a rhythmic spectrum. In research of the rhythmic spectrum, it is accidentally found by the inventor that a daytime rhythmic spectrum with a high visual effect and a high rhythmic stimulation effect or a nighttime rhythmic spectrum with a high visual effect and a low rhythmic stimulation effect can be obtained by performing color mixing on a white LED channel, a blue LED channel, a green LED channel and a blue-green LED channel and simply adjusting a luminous flux (or flux) of each channel. Therefore, the present disclosure is provided.

Specifically, a method for modulating a rhythmic spectrum in the present disclosure includes the following steps:
S1: A white LED, a blue LED, a green LED and a blue-green LED are provided on a substrate.

The white LED is a common white LED in existing markets. The white LED has a color temperature of 2,600-3,300 K. Exemplarily, the color temperature is 2,650 K, 2,700 K, 2,750 K, 2,800 K, 2,900 K, 3,000 K, 3,100 K or 3,200 K, but is not limited to this. Preferably, the white LED has the color temperature of 2,700 K.

The blue LED has a peak wavelength of 450-465 nm. Exemplarily, the peak wavelength is 450 nm, 454 nm, 456 nm, 458 nm, 460 nm, 462 nm or 464 nm, but is not limited to this. Preferably, the blue LED has the peak wavelength of 455 nm.

The green LED has a peak wavelength of 540-560 nm. Exemplarily, the peak wavelength is 541 nm, 545 nm, 548 nm, 552 nm, 555 nm, 557 nm or 559 nm, but is not limited to this. Preferably, the green LED has the peak wavelength of 550 nm.

The blue-green LED has a peak wavelength of 490-500 nm. Exemplarily, the peak wavelength is 492 nm, 494 nm, 496 nm or 498 nm, but is not limited to this. Preferably, the blue-green LED has the peak wavelength of 495 nm.

The substrate may be a light source plate, but is not limited to this. Preferably, the LEDs are evenly spaced.

S2: A luminous flux of each of the white LED, the blue LED, the green LED and the blue-green LED is adjusted to a preset range, thereby obtaining a rhythmic spectrum with a preset luminous flux.

Specifically, by adjusting the luminous flux, there are two modes, including a daytime mode and a nighttime mode. The specific process is described as follows:
Specifically, S2 includes:
S21: The luminous flux of each of the white LED, the blue LED, the green LED and the blue-green LED is adjusted.

Specifically, one LED is turned on individually, while other LEDs are turned off. A current is adjusted, an illuminance is measured with an illuminance meter, and the illuminance is used to calculate a corresponding luminous flux. After a required luminous flux is reached, the LED is turned off. The luminous flux of each LED is adjusted according to the above method. It is to be noted that there is no chronological order in adjustment.

Upon completion of the adjustment, a proportion of the luminous flux of each LED to a total luminous flux in the daytime lighting mode and the nighttime lighting mode is as follows:
The proportion of the luminous flux of each LED to the total luminous flux in the daytime lighting mode is as follows:
The luminous flux of the white LED accounts for 62.32-75.9% of the total luminous flux. Exemplarily, the proportion is 63.5%, 64.2%, 65.8%, 66.3%, 68.9%, 71.4%, 73.5%, 74.6% or 75.5%, but is not limited to this. Preferably, the luminous flux of the white LED accounts for 62.32-65.29% of the total luminous flux.

The luminous flux of the blue LED accounts for 1.74-1.91% of the total luminous flux. Exemplarily, the proportion is 1.75%, 1.77%, 1.8%, 1.83%, 1.85%, 1.87% or 1.89%, but is not limited to this. Preferably, the luminous flux of the blue LED accounts for 1.88-1.91% of the total luminous flux.

The luminous flux of the green LED accounts for 1.5-20.27% of the total luminous flux. Exemplarily, the proportion is 1.8%, 2.5%, 3.6%, 4.5%, 6.4%, 8.8%, 12.3%, 13.5%, 15.7%, 17.5% or 19.3%, but is not limited to this. Preferably, the luminous flux of the green LED accounts for 16.17-20.27% of the total luminous flux.

Preferably, the luminous flux of the blue-green LED accounts for 15.5-22.8% of the total luminous flux. Exemplarily, the proportion is 16.3%, 17.5%, 18.3%, 19.6%, 20.3%, 20.8%, 21.4% or 22.3%, but is not limited to this. Preferably, the luminous flux of the blue-green LED accounts for 15.5-16.66% of the total luminous flux.

The LEDs with the above luminous fluxes cooperate to form the rhythmic spectrum with the high rhythmic stimulation level and the high CRI.

The proportion of the luminous flux of each LED to the total luminous flux in the nighttime lighting mode is as follows:
The luminous flux of the white LED accounts for 61.8-69.3% of the total luminous flux. Exemplarily, the proportion is 62.3%, 63.4%, 64.8%, 65.3%, 66.7%, 66.9%, 67.5%, 68.6% or 69.1%, but is not limited to this. Preferably, the luminous flux of the white LED accounts for 61.83-66.35% of the total luminous flux.

The luminous flux of the blue LED accounts for 0.72-0.88% of the total luminous flux. Exemplarily, the proportion is 0.75%, 0.77%, 0.79%, 0.83%, 0.85% or 0.86%, but is not limited to this. Preferably, the luminous flux of the blue LED accounts for 0.82-0.87% of the total luminous flux.

The luminous flux of the green LED accounts for 28.6-37.31% of the total luminous flux. Exemplarily, the proportion is 28.8%, 29.5%, 30.6%, 31.5%, 32.4%, 33.8%, 34.3%, 35.5%, 36.7% or 37.2%, but is not limited to this. Preferably, the luminous flux of the green LED accounts for 31.06-37.31% of the total luminous flux.

Preferably, the luminous flux of the blue-green LED accounts for 0-1.8% of the total luminous flux. Exemplarily, the proportion is 0%, 0.05%, 0.4%, 0.6%, 0.8%, 1.1%, 1.4% or 1.6%, but is not limited to this. Preferably, the luminous flux of the blue-green LED accounts for 0-1.77% of the total luminous flux.

Specifically, after the luminous flux of each LED is adjusted, all LEDs are turned on at the same time. Light from each LED is mixed to obtain the rhythmic spectrum in the daytime or nighttime lighting mode.

S22: The white LED, the blue LED, the green LED and the blue-green LED are turned on at the same time, thereby obtaining the rhythmic spectrum with the preset luminous flux.

Specifically, the luminous flux of the spectrum can depend on a specific lighting environment. Exemplarily, in an embodiment of the daytime lighting mode, the preset luminous flux is 250 lm, but is not limited to this. It is to be noted that while other conditions are unchanged, the luminous flux is positively correlated with an average illuminance. That is, while the luminous flux increases, the average illuminance increases. Through calculation, the preset luminous flux in the embodiment is 250 lm, which meets the average illuminance of more than 500 lx.

Specifically, the daytime rhythmic spectrum obtained with the method of the present disclosure has an EML value of 242.41-269.5, and a CRI Ra of 84.0-96.86. The strong rhythmic stimulation and the high CRI are achieved at the same time. Evidences show that when the EML value is more than 240, strong rhythmic stimulation is achieved. This makes a human body keep an excited state, thereby improving a learning efficiency and a working efficiency. The environment is particularly suitable for a classroom scene, a working scene, etc.

The nighttime rhythmic spectrum obtained with the method of the present disclosure has an EML value of <120, and a CRI Ra of >80. Evidences show that in case of the EML value <120, weak rhythmic stimulation is achieved. This effectively reduces an influence on secretion of melatonin, without disturbing the rhythm of the human body. The environment is particularly suitable for night classes, night working scenes and so on, and improves night insomnia of the human body due to the strong rhythmic stimulation.

The present disclosure is described below with reference to specific embodiments:

### Embodiment 1

The embodiment provides a method for modulating a rhythmic spectrum in a daytime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 62.32% of a total luminous flux. The luminous flux of the blue LED accounts for 1.91% of the total luminous flux. The luminous flux of the green LED accounts for 20.27% of the total luminous flux. The luminous flux of the blue-green LED accounts for 15.5% of the total luminous flux.

(3) The white LED, the blue LED, the green LED and the blue-green LED are turned on at the same time to obtain a daytime rhythmic spectrum.

### Embodiment 2

The embodiment provides a method for modulating a rhythmic spectrum in a daytime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 65.29% of a total luminous flux. The luminous flux of the blue LED accounts for 1.88% of the total luminous flux. The luminous flux of the green LED accounts for 16.17% of the total luminous flux. The luminous flux of the blue-green LED accounts for 16.66% of the total luminous flux.

(3) The white LED, the blue LED, the green LED and the blue-green LED are turned on at the same time to obtain a daytime rhythmic spectrum.

### Comparative Embodiment 1

The comparative embodiment provides a method for modulating a rhythmic spectrum in a daytime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 23.34% of a total luminous flux. The luminous flux of the blue LED accounts for 2.33% of the total luminous flux. The luminous flux of the green LED accounts for 74.12% of the total luminous flux. The luminous flux of the blue-green LED accounts for 0.21% of the total luminous flux.

(3) The white LED, the blue LED, the green LED and the blue-green LED are turned on at the same time to obtain a daytime rhythmic spectrum.

### Comparative Embodiment 2

The comparative embodiment provides a method for modulating a rhythmic spectrum in a daytime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 30.32% of a total luminous flux. The luminous flux of the blue LED accounts for 2.25% of the total luminous flux. The luminous flux of the green LED accounts for 64.49% of the total luminous flux. The luminous flux of the blue-green LED accounts for 2.94% of the total luminous flux.

(3) The white LED, the blue LED, the green LED and the blue-green LED are turned on at the same time to obtain a daytime rhythmic spectrum.

### Comparative Embodiment 3

The comparative embodiment provides a method for modulating a rhythmic spectrum in a daytime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 39.51% of a total luminous flux. The luminous flux of the blue LED accounts for 2.15% of the total luminous flux. The luminous flux of the green LED accounts for 51.8% of the total luminous flux. The luminous flux of the blue-green LED accounts for 6.54% of the total luminous flux.

(3) The white LED, the blue LED, the green LED and the blue-green LED are turned on at the same time to obtain a daytime rhythmic spectrum.

### Comparative Embodiment 4

The comparative embodiment provides a method for modulating a rhythmic spectrum in a daytime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 54.74% of a total luminous flux. The luminous flux of the blue LED accounts for 1.99% of the total luminous flux. The luminous flux of the green LED accounts for 30.75% of the total luminous flux. The luminous flux of the blue-green LED accounts for 12.52% of the total luminous flux.

(3) The white LED, the blue LED, the green LED and the blue-green LED are turned on at the same time to obtain a daytime rhythmic spectrum.

### Comparative Embodiment 5

A common 5,000 K white LED (with 5,730 white beads produced by the Shenzhen Hengyi Photoelectric Technology Co., Ltd.) is selected.

While the color temperature is 5,000 K and the average illuminance is 500 Ix, the rhythmic spectrum in the daytime lighting mode in each of Embodiments 1-2 and Comparative Embodiments 1-5 is tested, with specific results shown in a table below:

| | Ra | R9 | EML |
|---|---|---|---|
| Embodiment 1 | 93.25 | 56.55 | 242.41 |
| Embodiment 2 | 91.37 | 64.43 | 246.33 |
| Comparative Embodiment 1 | 80.11 | 46.69 | 190.82 |
| Comparative Embodiment 2 | 84.74 | 50.73 | 200.04 |
| Comparative Embodiment 3 | 90.74 | 57.02 | 212.2 |
| Comparative Embodiment 4 | 96.86 | 69.32 | 232.37 |
| Comparative Embodiment 5 | 92.5 | 74.0 | 211.3 |

### Embodiment 3

The embodiment provides a method for modulating a rhythmic spectrum in a nighttime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 66.35% of a total luminous flux. The luminous flux of the blue LED accounts for 0.82% of the total luminous flux. The luminous flux of the green LED accounts for 31.06% of the total luminous flux. The luminous flux of the blue-green LED accounts for 1.77% of the total luminous flux.

(3) The white LED, the blue LED, the green LED and the blue-green LED are turned on at the same time to obtain a nighttime rhythmic spectrum.

### Embodiment 4

The embodiment provides a method for modulating a rhythmic spectrum in a nighttime lighting mode, including the following steps:
(1) A white LED, a blue LED, a green LED and a blue-green LED are provided on a light source plate.

The white LED has a color temperature of 2,700 K, the blue LED has a peak wavelength of 455 nm, and the green LED has a peak wavelength of 550 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 61.83% of a total luminous flux. The luminous flux of the blue LED accounts for 0.87% of the total luminous flux. The luminous flux of the green LED accounts for 37.31% of the total luminous flux.

(3) The white LED, the blue LED and the green LED are turned on at the same time to obtain a nighttime rhythmic spectrum.

### Comparative Embodiment 6

The comparative embodiment provides a method for modulating a rhythmic spectrum in a nighttime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 88.09% of a total luminous flux. The luminous flux of the blue LED accounts for 0.59% of the total luminous flux. The luminous flux of the green LED accounts for 1.01% of the total luminous flux. The luminous flux of the blue-green LED accounts for 10.31% of the total luminous flux.

(3) The white LED, the blue LED, the green LED and the blue-green LED are turned on at the same time to obtain a nighttime rhythmic spectrum.

### Comparative Embodiment 7

The comparative embodiment provides a method for modulating a rhythmic spectrum in a nighttime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 76.06% of a total luminous flux. The luminous flux of the blue LED accounts for 0.72% of the total luminous flux. The luminous flux of the green LED accounts for 17.63% of the total luminous flux. The luminous flux of the blue-green LED accounts for 5.59% of the total luminous flux.

(3) The white LED, the blue LED, the green LED and the blue-green LED are turned on at the same time to obtain a nighttime rhythmic spectrum.

### Comparative Embodiment 8

The comparative embodiment provides a method for modulating a rhythmic spectrum in a nighttime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 74.18% of a total luminous flux. The luminous flux of the blue LED accounts for 0.74% of the total luminous flux. The luminous flux of the green LED accounts for 20.24% of the total luminous flux. The luminous flux of the blue-green LED accounts for 4.85% of the total luminous flux.

(3) The white LED, the blue LED, the green LED and the blue-green LED are turned on at the same time to obtain a nighttime rhythmic spectrum.

### Comparative Embodiment 9

The comparative embodiment provides a method for modulating a rhythmic spectrum in a nighttime lighting mode, including the following steps:
(1) A white LED, a blue LED, a green LED and a blue-green LED are provided on a light source plate.

The white LED has a color temperature of 2,700 K, the blue LED has a peak wavelength of 455 nm, the green LED has a peak wavelength of 550 nm, and the blue-green LED has a peak wavelength of 495 nm.

(2) A luminous flux of each of the white LED, the blue LED, the green LED and the blue-green LED is adjusted. When the luminous flux of one LED is adjusted, other LEDs are turned off.

Specifically, the luminous flux of the white LED accounts for 70.82% of a total luminous flux. The luminous flux of the blue LED accounts for 0.78% of the total luminous flux. The luminous flux of the green LED accounts for 24.89% of the total luminous flux. The luminous flux of the blue-green LED accounts for 3.53% of the total luminous flux.

(3) The white LED, the blue LED, the green LED and the blue-green LED are turned on at the same time to obtain a nighttime rhythmic spectrum.

### Comparative Embodiment 10

A common 3,500K white LED (with 5,730 white beads produced by the Shenzhen Hengyi Photoelectric Technology Co., Ltd.) is selected.

While the color temperature is 3,500 K and the average illuminance is 300 Ix, the rhythmic spectrum in the nighttime lighting mode in each of Embodiments 3-4 and Comparative Embodiments 6-10 is tested, with specific results shown in a table below:

| | Ra | R9 | EML |
|---|---|---|---|
| Embodiment 3 | 91.1 | 63.1 | 119.9 |
| Embodiment 4 | 87 | 53.7 | 115.1 |
| Comparative Embodiment 6 | 94.21 | 61.32 | 142.94 |
| Comparative Embodiment 7 | 96.31 | 58.45 | 130.2 |
| Comparative Embodiment 8 | 95.22 | 66.63 | 128.2 |
| Comparative Embodiment 9 | 93.09 | 72.09 | 124.64 |
| Comparative Embodiment 10 | 93.0 | 72.0 | 147.5 |

The above are merely preferred implementations of the present disclosure. It should be noted that a person of ordinary skill in the art may further make several improvements and modifications without departing from the principle of the present disclosure, but such improvements and modifications should be deemed as falling within the protection scope of the present disclosure.

## Claims

1. A method for modulating a rhythmic spectrum, comprising:
(1) providing a white light-emitting diode, LED, a blue LED, a green LED and a blue-green LED on a substrate; and
(2) adjusting a luminous flux of each of the white LED, the blue LED, the green LED and the blue-green LED to a preset range, thereby obtaining a rhythmic spectrum with a preset luminous flux,
wherein the luminous flux of the white LED accounts for 62.32-75.9% of the preset luminous flux, the luminous flux of the blue LED accounts for 1.74-1.91% of the preset luminous flux, the luminous flux of the green LED accounts for 1.5-20.27% of the preset luminous flux, and the luminous flux of the blue-green LED accounts for 15.5-22.8% of the preset luminous flux; or the luminous flux of the white LED accounts for 61.8-69.3% of the preset luminous flux, the luminous flux of the blue LED accounts for 0.72-0.88% of the preset luminous flux, the luminous flux of the green LED accounts for 28.6-37.31% of the preset luminous flux, and the luminous flux of the blue-green LED accounts for 0-1.8% of the preset luminous flux.

2. The method for modulating a rhythmic spectrum according to claim 1, wherein step (2) comprises:
(2.1) adjusting the luminous flux of each of the white LED, the blue LED, the green LED and the blue-green LED, wherein when the luminous flux of one LED is adjusted, other LEDs are turned off; and
(2.2) turning on the white LED, the blue LED, the green LED and the blue-green LED at the same time, thereby obtaining the rhythmic spectrum with the preset luminous flux.

3. The method for modulating a rhythmic spectrum according to claim 1, wherein the white LED has a color temperature of 2,600-3,300 K, and the blue LED has a peak wavelength of 450-465 nm.

4. The method for modulating a rhythmic spectrum according to claim 1, wherein the green LED has a peak wavelength of 540-560 nm, and the blue-green LED has a peak wavelength of 490-500 nm.

5. The method for modulating a rhythmic spectrum according to any one of claims 1 to 4, wherein the white LED has the color temperature of 2,700 K, and the blue LED has the peak wavelength of 455 nm.

6. The method for modulating a rhythmic spectrum according to claim 5, wherein the green LED has the peak wavelength of 550 nm, and the blue-green LED has the peak wavelength of 495 nm.

7. The method for modulating a rhythmic spectrum according to claim 1, wherein the luminous flux of the white LED accounts for 62.32-65.29% of the preset luminous flux, the luminous flux of the blue LED accounts for 1.88-1.91% of the preset luminous flux, the luminous flux of the green LED accounts for 16.17-20.27% of the preset luminous flux, and the luminous flux of the blue-green LED accounts for 15.5-16.66% of the preset luminous flux.

8. The method for modulating a rhythmic spectrum according to claim 1, wherein the luminous flux of the white LED accounts for 61.83-66.35% of the preset luminous flux, the luminous flux of the blue LED accounts for 0.82-0.87% of the preset luminous flux, the luminous flux of the green LED accounts for 31.06-37.31% of the preset luminous flux, and the luminous flux of the blue-green LED accounts for 0-1.77% of the preset luminous flux.

9. The method for modulating a rhythmic spectrum according to claim 7, wherein the rhythmic spectrum has an equivalent melanopic lux, EML, of 242.41-269.5, and a color rendering index, CRI, Ra of 84.0-96.86.

10. The method for modulating a rhythmic spectrum according to claim 8, wherein the rhythmic spectrum has an EML of less than 120, and a CRI Ra of greater than or equal to 87.

## Patentansprüche

1. Verfahren zur Modulation eines rhythmischen Spektrums, umfassend:
(1) Bereitstellen einer weißen Leuchtdiode, LED, einer blauen LED, einer grünen LED und einer blau-grünen LED auf einem Substrat; und
(2) Anpassen eines Lichtstroms jeder der weißen LED, der blauen LED, der grünen LED und der blau-grünen LED auf einen voreingestellten Bereich, wodurch ein rhythmisches Spektrum mit einem voreingestellten Lichtstrom erhalten wird,
wobei der Lichtstrom der weißen LED 62,32-75,9 % des voreingestellten Lichtstroms beträgt, der Lichtstrom der blauen LED 1,74-1,91 % des voreingestellten Lichtstroms beträgt, der Lichtstrom der grünen LED 1,5-20,27 % des voreingestellten Lichtstroms beträgt und der Lichtstrom der blau-grünen LED 15,5-22,8 % des voreingestellten Lichtstroms beträgt; oder der Lichtstrom der weißen LED 61,8-69,3 % des voreingestellten Lichtstroms beträgt, der Lichtstrom der blauen LED 0,72-0,88 % des voreingestellten Lichtstroms beträgt, der Lichtstrom der grünen LED 28,6-37,31 % des voreingestellten Lichtstroms beträgt und der Lichtstrom der blau-grünen LED 0-1,8 % des voreingestellten Lichtstroms beträgt.

2. Verfahren zur Modulation eines rhythmischen Spektrums nach Anspruch 1, wobei Schritt (2) umfasst:
(2.1) Anpassen des Lichtstroms jeder der weißen LED, der blauen LED, der grünen LED und der blau-grünen LED, wobei beim Anpassen des Lichtstroms einer LED die anderen LEDs ausgeschaltet sind; und
(2.2) gleichzeitiges Einschalten der weißen LED, der blauen LED, der grünen LED und der blau-grünen LED, wodurch das rhythmische Spektrum mit dem voreingestellten Lichtstrom erhalten wird.

3. Verfahren zur Modulation eines rhythmischen Spektrums nach Anspruch 1, wobei die weiße LED eine Farbtemperatur von 2.600-3.300 K aufweist und die blaue LED eine Spitzenwellenlänge von 450-465 nm aufweist.

4. Verfahren zur Modulation eines rhythmischen Spektrums nach Anspruch 1, wobei die grüne LED eine Spitzenwellenlänge von 540-560 nm aufweist und die blau-grüne LED eine Spitzenwellenlänge von 490-500 nm aufweist.

5. Verfahren zur Modulation eines rhythmischen Spektrums nach einem der Ansprüche 1 bis 4, wobei die weiße LED die Farbtemperatur von 2.700 K aufweist und die blaue LED die Spitzenwellenlänge von 455 nm aufweist.

6. Verfahren zur Modulation eines rhythmischen Spektrums nach Anspruch 5, wobei die grüne LED die Spitzenwellenlänge von 550 nm aufweist und die blau-grüne LED die Spitzenwellenlänge von 495 nm aufweist.

7. Verfahren zur Modulation eines rhythmischen Spektrums nach Anspruch 1, wobei der Lichtstrom der weißen LED 62,32-65,29 % des voreingestellten Lichtstroms beträgt, der Lichtstrom der blauen LED 1,88-1,91 % des voreingestellten Lichtstroms beträgt, der Lichtstrom der grünen LED 16,17-20,27 % des voreingestellten Lichtstroms beträgt und der Lichtstrom der blau-grünen LED 15,5-16,66 % des voreingestellten Lichtstroms beträgt.

8. Verfahren zur Modulation eines rhythmischen Spektrums nach Anspruch 1, wobei der Lichtstrom der weißen LED 61,83-66,35 % des voreingestellten Lichtstroms beträgt, der Lichtstrom der blauen LED 0,82-0,87 % des voreingestellten Lichtstroms beträgt, der Lichtstrom der grünen LED 31,06-37,31 % des voreingestellten Lichtstroms beträgt und der Lichtstrom der blau-grünen LED 0-1,77 % des voreingestellten Lichtstroms beträgt.

9. Verfahren zur Modulation eines rhythmischen Spektrums nach Anspruch 7, wobei das rhythmische Spektrum einen äquivalenten melanopischen Lux, EML, von 242,41-269,5 und einen Farbwiedergabeindex, CRI, Ra von 84,0-96,86 aufweist.

10. Verfahren zur Modulation eines rhythmischen Spektrums nach Anspruch 8, wobei das rhythmische Spektrum einen EML von weniger als 120 und einen CRI Ra von größer oder gleich 87 aufweist.

## Revendications

1. Procédé de modulation d'un spectre rythmique, comprenant :
(1) la mise en œuvre d'une diode électroluminescente blanche, LED, d'une LED bleue, d'une LED verte et d'une LED bleu-vert sur un substrat ; et
(2) l'ajustement du flux lumineux de chacune de la LED blanche, de la LED bleue, de la LED verte et de la LED bleu-vert à une plage prédéfinie, permettant ainsi d'obtenir un spectre rythmique avec un flux lumineux prédéfini,
le flux lumineux de la LED blanche représentant 62,32 à 75,9 % du flux lumineux prédéfini, le flux lumineux de la LED bleue représentant 1,74 à 1,91 % du flux lumineux prédéfini, le flux lumineux de la LED verte représentant 1,5 à 20,27 % du flux lumineux prédéfini, et le flux lumineux de la LED bleu-vert représentant 15,5 à 22,8 % du flux lumineux prédéfini ; ou le flux lumineux de la LED blanche représentant 61,8 à 69,3 % du flux lumineux prédéfini, le flux lumineux de la LED bleue représentant 0,72 à 0,88 % du flux lumineux prédéfini, le flux lumineux de la LED verte représentant 28,6 à 37,31 % du flux lumineux prédéfini, et le flux lumineux de la LED bleu-vert représentant 0 à 1,8 % du flux lumineux prédéfini.

2. Procédé de modulation d'un spectre rythmique selon la revendication 1, dans lequel l'étape (2) comprend :
(2.1) l'ajustement du flux lumineux de chacune de la LED blanche, de la LED bleue, de la LED verte et de la LED bleu-vert, dans lequel, lors de l'ajustement du flux lumineux d'une LED, les autres LED sont éteintes ; et
(2.2) l'allumage simultané de la LED blanche, de la LED bleue, de la LED verte et de la LED bleu-vert, permettant ainsi d'obtenir le spectre rythmique avec le flux lumineux prédéfini.

3. Procédé de modulation d'un spectre rythmique selon la revendication 1, dans lequel la LED blanche présente une température de couleur comprise entre 2 600 et 3 300 K, et la LED bleue présente une longueur d'onde de crête comprise entre 450 et 465 nm.

4. Procédé de modulation d'un spectre rythmique selon la revendication 1, dans lequel la LED verte présente une longueur d'onde de crête comprise entre 540 et 560 nm, et la LED bleu-vert présente une longueur d'onde de crête comprise entre 490 et 500 nm.

5. Procédé de modulation d'un spectre rythmique selon l'une quelconque des revendications 1 à 4, dans lequel la LED blanche présente une température de couleur de 2 700 K, et la LED bleue présente une longueur d'onde de crête de 455 nm.

6. Procédé de modulation d'un spectre rythmique selon la revendication 5, dans lequel la LED verte présente une longueur d'onde de crête de 550 nm, et la LED bleu-vert présente une longueur d'onde de crête de 495 nm.

7. Procédé de modulation d'un spectre rythmique selon la revendication 1, dans lequel le flux lumineux de la LED blanche représente 62,32 à 65,29 % du flux lumineux prédéfini, le flux lumineux de la LED bleue représente 1,88 à 1,91 % du flux lumineux prédéfini, le flux lumineux de la LED verte représente 16,17 à 20,27 % du flux lumineux prédéfini, et le flux lumineux de la LED bleu-vert représente 15,5 à 16,66 % du flux lumineux prédéfini.

8. Procédé de modulation d'un spectre rythmique selon la revendication 1, dans lequel le flux lumineux de la LED blanche représente 61,83 à 66,35 % du flux lumineux prédéfini, le flux lumineux de la LED bleue représente 0,82 à 0,87 % du flux lumineux prédéfini, le flux lumineux de la LED verte représente 31,06 à 37,31 % du flux lumineux prédéfini, et le flux lumineux de la LED bleu-vert représente 0 à 1,77 % du flux lumineux prédéfini.

9. Procédé de modulation d'un spectre rythmique selon la revendication 7, dans lequel le spectre rythmique présente un lux mélanopique équivalent, EML, de 242,41 à 269,5, et un indice de rendu des couleurs, CRI, Ra de 84,0 à 96,86.

10. Procédé de modulation d'un spectre rythmique selon la revendication 8, dans lequel le spectre rythmique présente un EML inférieur à 120, et un CRI Ra supérieur ou égal à 87.
